# EUROPEAN PATENT APPLICATION

(11) **EP 2 130 440 A1**
(43) Date of publication of application: **09.12.2009**
(21) Application number: 08157759.5
(22) Date of filing: 06.06.2008
(51) Int. Cl.: A23L 1/03, A23C 3/08, A23L 3/3463

(54) **Inhibiting E. sakazakii growth**

(71) Applicant: N.V. Nutricia, 2712 HM Zoetermeer (NL)
(72) Inventor: Stahl, Bernd, 61191 Rosbach (DE); Fischer, Matthias, 63225 Langen (DE)
(74) Representative: Swinkels, Bart Willem

(57) **Abstract**

The present invention concerns the the use of uronic acid saccharide for preventing growth of *Enterobacter sakazakii.*

## Description

### FIELD OF THE INVENTION

The present invention relates to methods for preventing bacterial growth in products and treatment and/or prevention of infection, particularly in infants.

### BACKGROUND OF THE INVENTION

*Enterobacter sakazakii* (*E.sakazakii*) can cause serious infections, particularly in infants. Hence it is highly desirable to provide infant nutrition products with low *Enterobacter sakazakii* counts.

WO 2005049813 relates to phages isolates having a strong lytic activity against *Enterobacter sakazakii* strains and their use as anti-microbial agent in food products, in particular infant formula, and for sanitation of factory environments. The document also discloses food compositions and anti-microbial agents prepared thereof.

WO2007046698 relates to the use of a composition comprising non-digestible oligosaccharide for the manufacture of a composition for enteral administration to an infant delivered via caesarean section, particularly to the use of a composition comprising non-digestible oligosaccharides for the manufacture of a composition for (i) treatment and/or prevention of infection in infants delivered via caesarean section, wherein the infection is selected from the group consisting of Escherichia coli infection, Streptococcus infection, Clostridium infection, Bacillus infection, Pseudomonas infection, Enterobacter infection, Klebsiella infection, Acinetobacter infection, Proteus infection and Aeromonas infection.

### SUMARY OF THE INVENTION

The present inventors recognized that it is not only important to produce *E.sakazakii* free infant formula, it is also particularly important to prevent growth of *E.sakazakii* in the ready to feed (liquid) formula. For example, when a powdered infant formula is reconstituted with water, it may in contrast to the preparation instruction, be left standing for some time or stored, for example in a refrigerator. *E.sakazakii* can grow very rapidly in reconstituted infant formulae kept at room temperature. The microorganism is particularly well adapted to growth at temperatures around 37 - 44°C. Hence, under these circumstances *E.sakazakii* may multiply and reach unacceptable levels. Hence it is desirable to prevent growth in liquid products.

The present inventors found that pectin and pectin derived carbohydrates can suitably be used to prevent *Enterobacter sakazakii* growth or proliferation in a composition. It was found that *E.sakazakii* growth was significantly inhibited in liquid nutritional compositions which contain pectin derived carbohydrates.

Because of the inhibitory effects of the pectin degradation product on the proliferation *of E.sakazakii,* the saccharide can also be used to prevent growth of *E.sakazakii* in the oro-gastro-intestinal tract, and thereby reduce the incidence of *E.sakazakii* infection in mammals, particularly humans.

### DETAILED DESCRIPTION OF PREFERED EMBODIMENTS

The present invention relates to the use of acidic saccharides, preferably uronic acid saccharide for preventing growth of *Enterobacter sakazakii,* preferably for preventing growth of *Enterobacter sakazakii* in a composition. The present invention also relates to a method for preventing growth of *Enterobacter sakazakii,* preferably for preventing growth of *Enterobacter sakazakii* in a composition, said method comprising including uronic acid saccharide in a composition. Preferably said composition is a food product. Preferably said composition is suitable for administration to a mammal, preferably an infant.

In a further aspect, the present invention relates to (i) the use of uronic acid saccharide for treatment and/or prevention of *E.sakazakii* infection in a mammal; (ii) a method for treatment and/or prevention of *E.sakazakii* infection in a mammal, said method comprising administering uronic acid saccharide to said mammal. Also the invention relates to the use of a composition comprising uronic acid oligosaccharide for the manufacture of a composition for the treatment and/or prevention of *E.sakazakii* infection in a mammal. Also the invention relates to uronic acid oligosaccharide for use in the treatment and/or prevention of *E.sakazakii* infection in a mammal.

### Enterobacter sakazakii

*Enterobacter sakazakii* (*E.sakazakii*) is a Gram-negative rod-shaped pathogenic bacterium of the genus *Enterobacter. E.sakazakii* is an opportunistic pathogen that can cause infections such as necrotizing enterocolitis, bacteraemia, meningitis and brain abscess/lesions (Iversen et al. (2007) BMC Evol Biol 7: 64).

### Growth inhibition

The present invention relates to the use of uronic acid saccharide for preventing growth of *E.sakazakii.* Particularly, the present invention provides a reduced *E.sakazakii* bacterial count in a liquid composition comprising uronic acid saccharide compared to the same composition without the uronic acid saccharide, particularly if some time has passed after preparing the composition. According to the present invention growth is significantly reduced if the number of colony forming units (cfu) of *E.sakazakii* per ml is preferably at least 10 times, more preferably 100 times, most preferably at least 1000 times, less in a liquid composition comprising uronic acid saccharide compared to the same composition without the uronic acid saccharide after 24 h of incubation at room temperature.

The present invention relates to the use of uronic acid saccharide for preventing growth *of E.sakazakii,* particularly in a composition comprising protein, fat and carbohydrates, preferably a liquid composition. Hence, the uronic acid saccharide is preferably used in liquid food products, particularly infant formula. In the present composition, preferably the lipid component provides 5 to 50% of the total calories, the protein component provides 5 to 50% of the total calories, the carbohydrate component provides 15 to 90% of the total calories. Preferably, in the present composition the lipid component provides 35 to 50% of the total calories, the protein component provides 7.5 to 12.5% of the total calories, and the carbohydrate component provides 40 to 55% of the total calories. For calculation of the % of total calories for the protein component, the total of energy provided by the proteins, peptides and amino acids needs to be taken into account. The protein component used in the nutritional preparation are preferably selected from the group consisting of non-human animal proteins (preferably milk proteins), vegetable proteins (preferably soy protein and/or rice protein), hydrolysates thereof, free amino acids and mixtures thereof. The composition preferably contains at least one digestible carbohydrates selected from the group consisting of sucrose, lactose, glucose, fructose, corn syrup solids, starch and maltodextrins, and mixtures thereof, more preferably lactose. Hence, the composition in liquid form preferably has a caloric density between 0.1 and 2.5 kcal/ml, even more preferably a caloric density of between 0.5 and 1.5 kcal/ml, most preferably between 0.6 and 0.8 kcal/ml.

The composition preferably comprises between 0.01 and 10 g uronic acid saccharide with a DP of 2 to 250, preferably a DP of 2 to 100, per 100 g dry weight of the present composition, more preferably between 0.05 and 6 g, even more preferably 0.2 to 2 g per 100 g dry weight. The composition preferably comprises between 0.01 and 10 g galacturonic acid oligosaccharide with a DP of 2 to 250, preferably a DP of 2 to 100, per 100 g dry weight of the present composition, more preferably between 0.05 and 6 g, even more preferably 0.2 to 2 g. The composition preferably comprises between 0.01 and 10 g pectin degradation product with a DP of 2 to 250, preferably a DP of 2 to 100, per 100 g dry weight of the present composition, more preferably between 0.05 and 6 g, even more preferably 0.2 to 2 g.

Thus, in one embodiment the invention concerns the use of uronic acid saccharide for preventing growth of *Enterobacter sakazakii* in a liquid composition, said composition comprising lipid, protein and carbohydrate wherein the lipid provides 5 to 50% of the total calories, the protein provides 5 to 50% of the total calories, the carbohydrate provides 15 to 90% of the total calories and said composition comprising between 0.01 and 10 g uronic acid saccharide with a DP of 2 to 250 per 100 g dry weight of said composition.

### Infection inhibition

The present invention also relates to the use of uronic acid saccharide in the preparation of a composition for the treatment and/or prevention of *E.sakazakii* infection in mammal. The specific growth inhibitory effects as presently found can also be advantageously used in humans. Oral ingestion of the uronic acid oligosaccharide prevents growth of *E.sakazakii* in the intestine, thereby reducing the incidence and severity *of E.sakazakii* infection.

*E.sakazakii* infection plays a role in the development of neonatal meningitis and necrotizing enterocolitis. Hence in a preferred embodiment, the present invention relates to the use of uronic acid saccharide in the preparation of a composition for the treatment and/or prevention of neonatal meningitis and/or necrotizing enterocolitis, more preferably E.sakazakii induced neonatal meningitis and/or E.sakazakii necrotizing enterocolitis. In particular the present invention relates to uronic acid saccharide for use in the treatment and/or prevention of *E.sakazakii* infection in a mammal, neonatal meningitis and/or necrotizing enterocolitis. Preferably the present invention relates to the treatment and/or prevention of a human infant with the age between 0 and 2 years.

Preferably the uronic acid saccharide is administered in combination with or mixed to a nutritional composition. Preferred nutritional compositions are described hereinabove. In one embodiment of the present invention the uronic acid saccharide is in a liquid composition, said composition comprising lipid, protein and carbohydrate wherein the lipid provides 5 to 50% of the total calories, the protein provides 5 to 50% of the total calories, the carbohydrate provides 15 to 90% of the total calories and said composition comprising between 0.01 and 10 g uronic acid saccharide with a DP of 2 to 250, preferably a DP of 2 to 100, per 100 g dry weight of said composition.

### Uronic acid saccharide

The term uronic acid saccharide as used in the present invention refers to a saccharide comprising uronic acid monosaccharide units, preferably at least 25%, preferably at least 50%, of the monosaccharide units present in the saccharide is a uronic acid. Preferably the present uronic acid saccharide comprising uronic acid monosaccharide units is one selected from the group consisting of guluronic acid, iduronic acid, riburonic acid, mannuronic acid, galacturonic acid and glucuronic acid. In a preferred embodiment the uronic acid saccharide comprises galacturonic acid, preferably at least 50% galacturonic acid based on total uronic acid units in the uronic acid saccharide. Preferably the uronic acid saccharide is pectin, alginate, a degradation product of pectin or a degradation product of alginate, most preferably a degradation product of pectin. Good *E.sakazakii* inhibitory effects have been achieved with pectin degradation products.

Preferably the pectin degradation product is a pectin hydrolysate (prepared by hydrolysis) and/or pectin lyasate (prepared by beta-elimination). The pectin degradation product is preferably prepared from fruit or vegetable pectin, more preferably from apple pectin, citrus pectin and/or sugar beet pectin. The pectin degradation product is preferably prepared with lyases and/or variations of the temperature and pressure, more preferably by beta-elimination. The pectin degradation product is preferably a pectin lyasate.

Preferably the present composition comprises uronic acid saccharide with a degree of polymerization (DP) of 2 to 250, more preferably a DP of 2 to 100, even more preferably a DP of 2 to 50, most preferably a DP of 2 to 20. Preferably the present composition comprises between 25 and 100 wt.%, more preferably between 50 and 100 wt.% uronic acid saccharide with a DP of 2 to 250 based on total weight of uronic acid in the composition, more preferably a DP of 2 to 100, even more preferably a DP of 2 to 50, most preferably a DP of 2 to 20. Preferably the uronic acid saccharide is a degradation product of pectin.

In a preferred embodiment at least one of the terminal hexuronic acid units of the uronic acid saccharide has a double bond, which is preferably situated between the C₄ and C₅ position of the terminal hexuronic acid unit. Preferably at least 5%, more preferably at least 10%, even more preferably at least 25% of the terminal hexuronic acid units of the uronic acid saccharide is an unsaturated hexuronic acid unit. As each individual uronic acid saccharide preferably comprises only one unsaturated terminal hexuronic acid unit, preferably less than 50% of the terminal hexuronic acid units is an unsaturated hexuronic acid unit (i.e. comprises a double bond).

The uronic acid saccharide can be derivatised. The uronic acid saccharide may be methoxylated and/or amidated. In a preferred embodiment the uronic acid saccharide is characterized by a degree of methoxylation above 20%, preferably above 30%, or above 50% or even above 70%. As used herein, "degree of methoxylation" (also referred to as DE or "degree of esterification") is intended to mean the extent to which free carboxylic acid groups contained in the uronic acid saccharide have been esterified (e.g. by methylation). In another embodiment the uronic acid saccharide has a degree of methylation above 20%, preferably above 30%, or above 50% or even above 70%.

Acidic saccharide in the cotnext of this invention is defined a saccharide that is negatively charged wheb dissolved in water at neutral pH, preferably having a negativelty charged carboxylic acid.

### EXAMPLES

### Anti-pathogenic effect of uronic acid saccharide

The inhibition of microbial growth by uronic acid saccharide, in particular pectin-derived oligosaccharide, was investigated. For the present investigation pectin lyasate with a degree of methylation of 36% was used at a concentration of 14 mg/ml.

*Enterobacter sakazakii* (DSM 4485) was diluted with a solution of NaCL-peptone and incubated overnight. The initial inoculation of the samples amounted to 10 cfu/measuring unit in BiMedia 001B (Sy-Lab, Austria) as basic culture medium enriched with uronic acid saccharide. The samples were analyzed at 0, 6 and 24 hours after start of incubation by conventional plating method. The results of bacterial growth are given in the following table 1.

**Table 1: Bacterial growth**

| | germination number of micororganism (cfu/ml) |
|---|---|
| | *E.sakazakii* (24 h) |
| Control | 1.10⁹ |
| Pectin lyasate | 0.5 |

The table above shows the unexpected efficient and unexpected specific inhibition by uronic acid saccharide *of E.sakazakii* which demonstrates the utility of the acid saccharide for preventing growth of the microorganism in compositions and is also indicative for the beneficial use in the treatment and/or prevention of *E.sakakzakii* infection.

## Claims

1. Use ofuronic acid saccharide for preventing growth of *Enterobacter sakazakii.*

2. Use according to claim 1, for preventing growth of *Enterobacter sakazakii* in a liquid composition, said composition comprising lipid, protein and carbohydrate wherein the lipid provides 5 to 50% of the total calories, the protein provides 5 to 50% of the total calories, the carbohydrate provides 15 to 90% of the total calories and said composition comprising between 0.01 and 10 g uronic acid saccharide with a DP of 2 to 250 per 100 g dry weight of said composition.

3. Use of uronic acid saccharide in the preparation of a composition for the treatment and/or prevention of (i) *Enterobacter sakazakii* infection in a mammal; (ii)neonatal meningitis; and/or (iii) necrotizing enterocolitis.

4. Use according to claim 3, wherein the treatment and/or prevention is in a human infant with the age between 0 and 2 years.

5. Use according to claim 3 or 4, wherein the uronic acid saccharide is in a liquid composition, said composition comprising lipid, protein and carbohydrate wherein the lipid provides 5 to 50% of the total calories, the protein provides 5 to 50% of the total calories, the carbohydrate provides 15 to 90% of the total calories and said composition comprising between 0.01 and 10 g uronic acid saccharide with a DP of 2 to 250 per 100 g dry weight of said composition.

6. Use according to any one of the preceding claims, wherein the uronic acid saccharide is a pectin degradation product.
